# EUROPEAN PATENT APPLICATION

(11) **EP 4 393 305 A1**
(43) Date of publication of application: **03.07.2024**
(21) Application number: 22861422.8
(22) Date of filing: 24.08.2022
(51) Int. Cl.: A01N 25/00, A01N 33/04, A01N 59/08, A01P 1/00, A01P 3/00, A61K 8/20, A61K 8/41, A61K 8/73, A61K 8/81, A61K 33/20, A61L 9/01, A61P 1/02, A61P 17/00, A61Q 11/00, A61Q 19/00, B01J 31/02

(54) **COMPOSITION**

(30) Priority: 25.08.2021 JP 2021137556
(71) Applicant: Earth Corporation, Tokyo 101-0048 (JP)
(72) Inventor: OTA Manami, Ako City, Hyogo 6780192 (JP); ASAI Rika, Ako City, Hyogo 6780192 (JP)
(74) Representative: Grünecker Patent- und Rechtsanwälte PartG mbB
(86) International application number: PCT/JP2022/031963
(87) International publication number: WO 2023/027134

(57) **Abstract**

An object of the present invention is to provide a novel form of a composition containing a radical generation source and a radical generation catalyst. The composition in the present invention contains a radical generation catalyst (a), a radical generation source (b), a thickener (c), and water (d).

## Description

### TECHNICAL FIELD

The present invention relates to a composition, and more particularly to a composition that generates radicals.

### BACKGROUND ART

Radicals are rich in reactivity and have strong oxidizing power, so that the radicals are important chemical species widely used. For example, when bacteria or viruses come into contact with radicals, the bacteria or viruses disappear (also referred to as inactivation) by oxidizing power of the radicals. Therefore, effects such as sterilization and deodorization can be obtained, and the radicals are used in various fields such as medical care, hygiene, food processing, and water treatment.

As a sterilization technique using radicals, for example, Patent Literature 1 proposes a drug including a radical generation catalyst and a radical generation source, in which the radical generation catalyst contains one or both of: at least one or both of ammonium and a salt thereof; and a substance having at least one of Lewis acidity and Bronsted acidity.

### CITATION LIST

### PATENT LITERATURE

Patent Literature 1: JP2017-109978A

### SUMMARY OF INVENTION

### TECHNICAL PROBLEM

The drug described in Patent Literature 1 is mixed with water, an organic solvent, or the like, and the mixture is sprayed or applied to an object, and used in the form of a liquid preparation.

In recent years, the formulation design has been diversified in order to obtain a suitable dosage form in accordance with an applied place of use, an applied purpose of use, an applied use, and the like, and in order to meet such diversified requirements, it is required to use a composition that generates radicals in a new dosage form.

Therefore, an object of the present invention is to provide a novel form of a composition containing a radical generation source and a radical generation catalyst.

### SOLUTION TO PROBLEM

The present invention is achieved by the following (1) to (7).
(1) A composition containing a radical generation catalyst (a), a radical generation source (b), a thickener (c), and water (d).
(2) The composition according to the above (1), in which the radical generation catalyst is a Lewis acid having a Lewis acidity of 0.4 eV or more.
(3) The composition according to the above (1) or (2), in which the radical generation catalyst contains an ammonium salt represented by the following chemical formula (I). 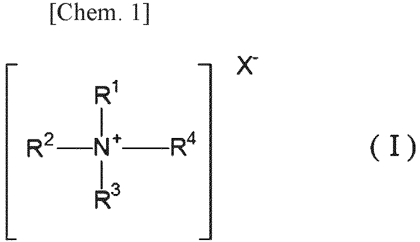
   In the chemical formula (I), R¹, R², R³, and R⁴ are the same or different from each other, each represents a hydrogen atom or an alkyl group, and may contain an ether bond, a carbonyl group, an ester bond, an amide bond, or an aromatic ring, and X⁻ represents an anion.
(4) The composition according to the above (3), in which the ammonium salt represented by the chemical formula (I) is an ammonium salt represented by the following chemical formula (II). 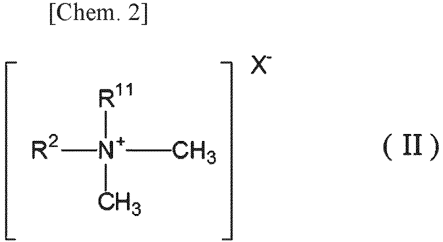
   In the chemical formula (II), R¹¹ represents an alkyl group having 5 to 40 carbon atoms and may contain an ether bond, a carbonyl group, an ester bond, an amide bond, or an aromatic ring, and R² and X⁻ are the same as those in the chemical formula (I).
(5) The composition according to the above (4), in which the ammonium salt represented by the chemical formula (II) is an ammonium salt represented by the following chemical formula (III). 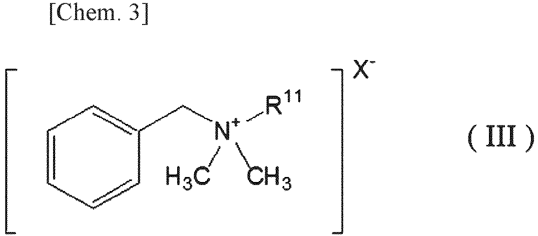
   In the chemical formula (III), R¹¹ and X⁻ are the same as those in the chemical formula (II).
(6) The composition according to the above (1), in which the radical generation source is at least one selected from the group consisting of a halous acid, a halite ion, and a halite.
(7) The composition according to the above (1), which is neutral or alkaline.

### ADVANTAGEOUS EFFECTS OF INVENTION

According to the present invention, a new viscous composition such as gel that generates radicals can be provided. The composition according to the present invention can be easily handled, and for example, can be easily applied to a target.

### BRIEF DESCRIPTION OF DRAWINGS

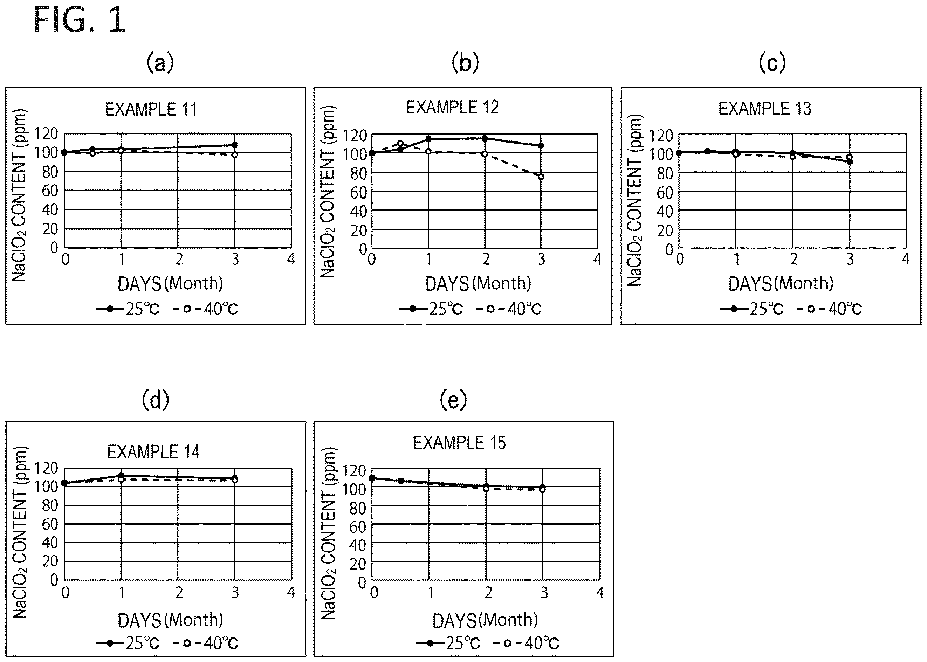

[FIG. 1] FIG. 1 is a diagram showing results of a temporal test of a content of sodium chlorite in sample compositions in Test Example 3, and (a) of FIG. 1 is that of Example 11, (b) of FIG. 1 is that of Example 12, (c) of FIG. 1 is that of Example 13, (d) of FIG. 1 is that of Example 14, and (e) of FIG. 1 is that of Example 15.

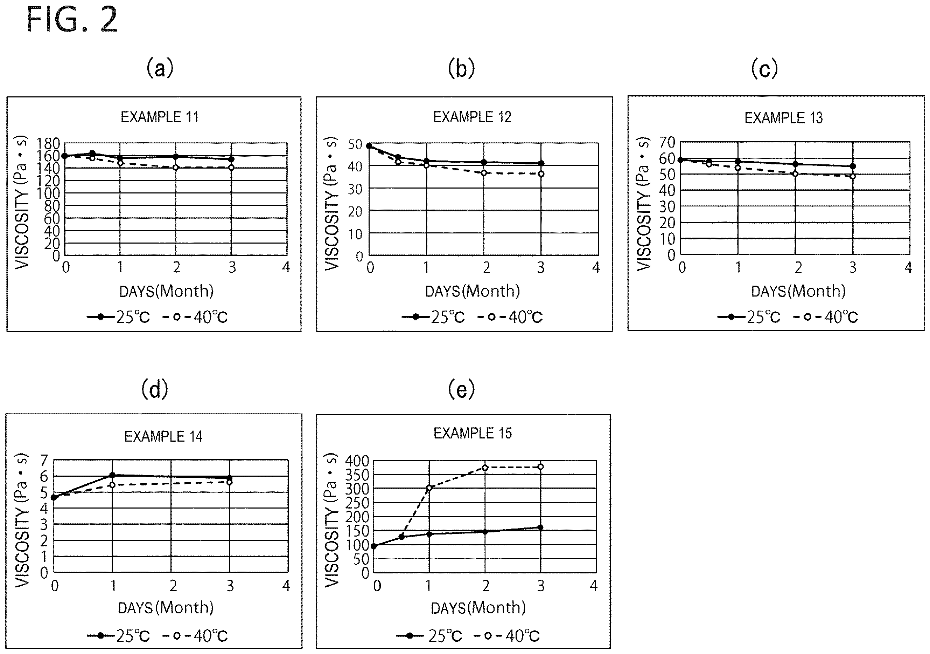

[FIG. 2] FIG. 2 is a diagram showing results of a temporal test of a viscosity of sample compositions in Test Example 4, and (a) of FIG. 2 is that of Example 11, (b) of FIG. 2 is that of Example 12, (c) of FIG. 2 is that of Example 13, (d) of FIG. 2 is that of Example 14, and (e) of FIG. 2 is that of Example 15.

### DESCRIPTION OF EMBODIMENTS

Hereinafter, embodiments of the present invention are described in more detail.

In the present description, "mass" has the same meaning as "weight".

The composition in the present invention contains a radical generation catalyst (a), a radical generation source (b), a thickener (c), and water (d). Hereinafter, each component is described.

### <(a) Radical Generation Catalyst>

The radical generation catalyst contained in the composition according to the present invention (hereinafter may be referred to as "radical generation catalyst according to the present invention") is not particularly limited as long as it catalyzes generation of radicals from a radical generation source, and common compounds can be used. One kind of the radical generation catalysts may be used alone, or two or more kinds thereof may be used in combination.

In the present invention, a Lewis acid is preferably used as the radical generation catalyst, and a Lewis acid having a Lewis acidity of 0.4 eV or more is more preferable.

The upper limit of the Lewis acidity is not particularly limited, and in terms of the upper limit, the Lewis acidity is preferably 20 eV or less. The Lewis acidity can be measured by, for example, a method described in Ohkubo, K.; Fukuzumi, S. Chem. Eur. J., 2000, 6, 4532, J. AM. CHEM. SOC. 2002, 124, 10270-10271, or J. Org. Chem. 2003, 68, 4720-4726, and specifically can be measured by the following method.

### (Method For Measuring Lewis Acidity)

Acetonitrile (MeCN) containing cobalt tetraphenylporphyrin (CoTPP), saturated O₂, and a substance to be measured for the Lewis acidity (which is, for example, a cation of metal or the like and is represented by Mⁿ⁺ in the following reaction scheme (A)) in the following reaction scheme (A) is measured for a change in an ultraviolet-visible absorption spectrum at room temperature. From the obtained reaction rate constant (kcat), the ΔE value (eV) as an index of the Lewis acidity can be calculated. Strong Lewis acidity is exhibited as the value of kcat is increased. The Lewis acidity of an organic compound can also be estimated from an energy level of the lowest unoccupied molecular orbital (LUMO) calculated by quantum chemical calculation. Strong Lewis acidity is exhibited as the value is increased on the positive side.

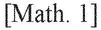

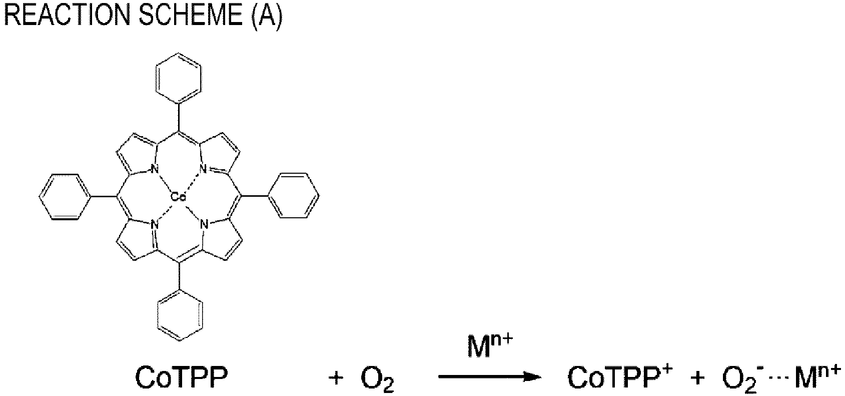

Examples of the reaction rate constant of CoTPP and oxygen in the presence of a Lewis acid, which is an index of the Lewis acidity measured (calculated) by the above measurement method, are shown below. In the following Table 1, numerical values represented by "k_{cat}, M⁻²s⁻¹" are CoTPP and oxygen in the presence of a Lewis acid. The numerical value represented by "LUMO, eV" is an energy level of LUMO.

### [Table 1]

**Table 1**

| | LUMO, eV | k_{cat}, M⁻²s⁻¹ |
|---|---|---|
| Benzethonium chloride | -4.12 | 0.24 |
| Benzalkonium chloride | -4.02 | 0.18 |
| Tetramethylammonium hexafluorophosphate | -3.58 | > 0.1 |
| Tetrabutyl ammonium hexafluorophosphate | -2.07 | > 0.1 |
| Ammonium hexafluorophosphate | -5.73 | 20 |

The radical generation catalyst according to the present invention is preferably ammonium having properties as a Lewis acid or a salt thereof. Such ammonium may be, for example, quaternary ammonium, or tertiary ammonium, secondary ammonium, primary ammonium, or ammonium.

Examples of the ammonium and the salt thereof include cationic surfactants, and among them, a quaternary ammonium-based cationic surfactant is preferable.

Examples of the quaternary ammonium-based cationic surfactant include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxitropium, carbachol, glycopyrronium, safranin, sinapine, tetraethylammonium bromide, hexadecyltrimethylammonium bromide, suxamethonium, sphingomyelin, denatonium, trigonelline, neostigmine, paraquat, pyridostigmine, phellodendrine, pralidoxime methiodide, betaine, betanin, bethanechol, betalain, lecithin, and cholines (choline chloride such as benzoyl choline chloride and lauroylcholine chloride hydrate, phosphocholine, acetylcholine, choline, dipalmitoylphosphatidylcholine, and choline bitartrate). One kind of these may be used alone, or two or more kinds thereof may be used in combination.

Here, in the present invention, the quaternary ammonium is not limited only to surfactants.

In the radical generation catalyst according to the present invention, the ammonium may be, for example, an ammonium represented by the following chemical formula (I).

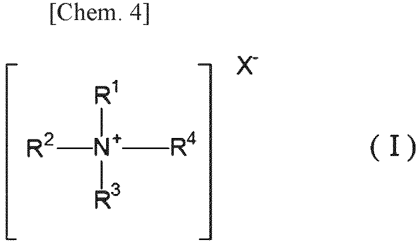

In the chemical formula (I), R¹, R², R³, and R⁴ are the same or different from each other, each represents a hydrogen atom or an alkyl group, and may contain an ether bond, a ketone (carbonyl group), an ester bond, an amide bond, or an aromatic ring, and X⁻ represents an anion.

The alkyl group is preferably a linear or branched alkyl group having 1 to 40 carbon atoms.

The ammonium represented by the chemical formula (I) is preferably an ammonium represented by the following chemical formula (II).

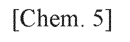

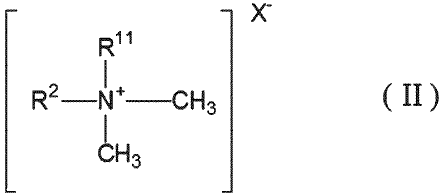

In the chemical formula (II), R¹¹ represents an alkyl group having 5 to 40 carbon atoms, and may contain an ether bond, a ketone (carbonyl group), an ester bond, an amide bond, or an aromatic ring, and R² and X⁻ are the same as those of the chemical formula (I).

In the chemical formula (II), R² is preferably a methyl group or a benzyl group. In the benzyl group, one or more hydrogen atoms on the benzene ring may be or may not be substituted with any substituent. Examples of any substituent include an alkyl group, an unsaturated aliphatic hydrocarbon group, an aryl group, a heteroaryl group, a halogen, a hydroxy group (-OH), a mercapto group (-SH), and an alkylthio group (-SR, where R represents an alkyl group).

The ammonium represented by the chemical formula (II) is preferably an ammonium represented by the following chemical formula (III).

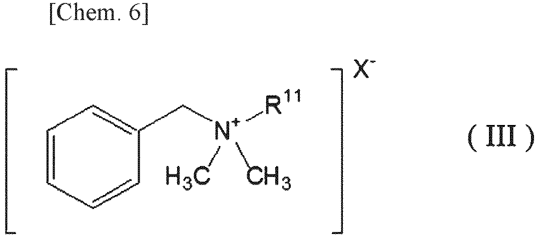

In the chemical formula (III), R¹¹ and X⁻ are the same as those in the chemical formula (II).

Specific examples of the ammonium represented by the chemical formula (I) include benzethonium chloride, benzalkonium chloride, hexadecyltrimethylammonium chloride, tetramethylammonium chloride, ammonium chloride, and tetrabutylammonium chloride. The ammonium is preferably at least one selected from the group consisting of them. Among them, benzethonium chloride represented by the formula (II) is particularly preferable.

Benzethonium chloride (Bzn⁺Cl⁻) can be represented by, for example, the following chemical formula (IV). In the formula (IV), Me represents a methyl group, and ^{t}Bu represents a tertiary butyl group.

The benzalkonium chloride can be represented, for example, as a compound represented by the chemical formula (III) in which R¹¹ represents an alkyl group having 8 to 18 carbon atoms and X⁻ represents chloride ions.

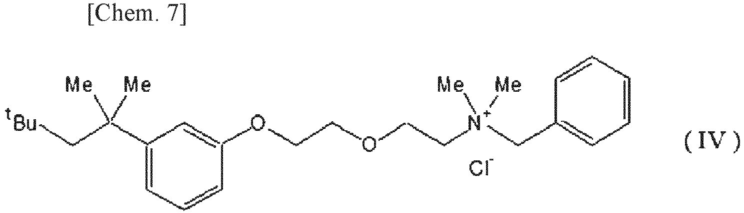

In the chemical formulae (I), (II), and (III), X' represents any anions and is not particularly limited. In addition, X' is not limited to monovalent anions, and may be anions of any valence such as divalent anions and trivalent anions. When the anions have plural charges such as divalent anions or trivalent anions, for example, the number of molecules of the ammonium (monovalent) in the chemical formulae (I), (II), and (III) is expressed as "number of molecules of anions" × "valence of anions" (for example, when the anion is divalent, the number of molecules of the ammonium (monovalent) is twice the number of molecules of anions). Examples of X⁻ include halogen ions (fluoride ions, chloride ions, bromide ions, and iodide ions), acetate ions, nitrate ions, and sulfate ions.

In the present invention, the ammonium may contain a plurality of ammonium structures (N⁺) in one molecule. Further, for example, the ammonium may form a dimer, a trimer, or the like by associating a plurality of molecules by π-electron interaction.

In the present invention, when isomers such as a tautomer or stereoisomer (for example, a geometric isomer, a conformational isomer, and an optical isomer) are present in a compound (for example, an organic ammonium or the like), any isomer can be used in the present invention unless otherwise specified.

When the compound (for example, the organic ammonium or the like) can form a salt, the salt may be an acid addition salt or a base addition salt. Further, an acid forming the acid addition salt may be an inorganic acid or an organic acid, and the base forming the base addition salt may be an inorganic base or an organic base. The inorganic acid is not particularly limited, and examples thereof include sulfuric acid, phosphoric acid, hydrofluoric acid, hydrochloric acid, hydrobromic acid, hydriodic acid, hypofluorous acid, hypochlorous acid, hypobromous acid, hypoiodous acid, fluorous acid, chlorous acid, bromous acid, iodous acid, fluoric acid, chloric acid, bromic acid, iodic acid, perfluoric acid, perchloric acid, perbromic acid, and periodic acid. The organic acid is also not particularly limited, and examples thereof include p-toluene sulfonic acid, methane sulfonic acid, oxalic acid, p-bromobenzenesulfonic acid, carbonic acid, succinic acid, citric acid, benzoic acid, and acetic acid. The inorganic base is not particularly limited, and examples thereof include ammonium hydroxide, alkali metal hydroxides, alkaline earth metal hydroxides, carbonates, and hydrogen carbonates. Specific examples of the inorganic base include sodium hydroxide, potassium hydroxide, potassium carbonate, sodium carbonate, sodium hydrogen carbonate, potassium hydrogen carbonate, calcium hydroxide, and calcium carbonate. The organic base is also not particularly limited, and examples thereof include ethanolamine, triethylamine, and tris(hydroxymethyl)amino methane. A method for producing these salts is not particularly limited, and for example, these salts can be produced by a method in which the acid or the base as described above is appropriately added to the compound by a common method.

In the present invention, a chain substituent (for example, a hydrocarbon group such as an alkyl group or an unsaturated aliphatic hydrocarbon group) may be linear or branched unless otherwise specified, and the number of carbon atoms thereof is not particularly limited and is preferably 1 to 40, 1 to 32, 1 to 24, 1 to 18, 1 to 12, 1 to 6, or 1 to 2 (2 or more in the case of the unsaturated hydrocarbon group) in this order. In the present invention, the number of ring members (the number of atoms constituting a ring) of a cyclic group (for example, an aryl group and a heteroaryl group) is not particularly limited, and is preferably 5 to 32, 5 to 24, 6 to 18, 6 to 12, or 6 to 10 in this order. When an isomer is present in a substituent or the like, any isomer may be used unless otherwise specified. For example, when simply referred to as a "naphthyl group", it may be a 1-naphthyl group or a 2-naphthyl group.

In the composition according to the present invention, a content of the radical generation catalyst is preferably 0.01 ppm by mass to 1500 ppm by mass. When a concentration of the radical generation catalyst in the composition is too low, the generation of radicals may be prevented, and the sterilization effect may not be obtained. The content of the radical generation catalyst of 1500 ppm by mass or less is preferable because safety can be ensured.

The content of the radical generation catalyst in the composition is more preferably 0.1 ppm by mass to 1000 ppm by mass, still more preferably 0.1 ppm by mass to 500 ppm by mass, particularly preferably 1 ppm by mass to 200 ppm by mass, and most preferably 1 ppm by mass to 100 ppm by mass.

From the viewpoint of preventing a situation in which the sterilization effect or the like cannot be obtained by the micelle formation, the concentration of the radical generation catalyst is preferably equal to or lower than a micelle limit concentration.

### <(b) Radical Generation Source>

Examples of the radical generation source include a halogen ion, a hypohalite ion, a halite ion, a halide ion, and a perhalate ion. It is preferable to contain at least one selected from the group consisting of these ions.

The radical generation source may contain, for example, an oxoacid or a salt thereof (for example, a halogen oxoacid or a salt thereof). Examples of the oxoacid include boric acid, carbonic acid, orthocarbonic acid, carboxylic acid, silicic acid, nitrous acid, nitric acid, phosphorous acid, phosphoric acid, arsenic, sulphurous acid, surfuric acide, sulfonic acid, sulfinic acid, chromic acid, dichromic acid, and permanganic acid. Examples of a halogen oxoacid include: chlorine oxoacids such as hypochlorous acid, chlorous acid, chloric acid, and perchloric acid; bromine oxoacids such as hypobromous acid, bromous acid, bromic acid, and perbromic acid; and iodine oxoacids such as hypoiodous acid, iodous acid, iodic acid, and periodic acid. Examples of the salts include alkali metal salts such as sodium and potassium.

Among them, it is more preferable that the radical generation source is at least one selected from the group consisting of a halous acid, a halite ion, and a halite, and it is still more preferable that the radical generation source is at least one selected from the group consisting of a chlorous acid, a chlorite ion, and a chlorite from the viewpoint that the reactivity with the radical generation catalyst is moderate and the reaction is easily controlled.

Specifically, sodium chlorite is preferable.

In the composition according to the present invention, a content of the radical generation source is preferably 0.01 ppm by mass to 1500 ppm by mass. When a concentration of the radical generation source in the composition is too low, the amount of radicals generated is too small, and thus the sterilization effect or the like may not be obtained. In addition, the higher the concentration of the radical generation source is, the better the sterilization effect is, but the concentration is preferably 1500 ppm by mass or less from the viewpoint of ensuring safety.

The content of the radical generation source in the composition is more preferably 1 ppm by mass to 1000 ppm by mass, still more preferably 10 ppm by mass to 500 ppm by mass, and particularly preferably 50 ppm by mass to 250 ppm by mass.

In the composition according to the present invention, a concentration ratio of the radical generation source to the radical generation catalyst (radical generation source/radical generation catalyst) in the composition is not particularly limited and can be appropriately set.

### <(c) Thickener>

As the thickener, a water-soluble thickener can be preferably used. Among them, nonionic thickeners and amphoteric thickeners are preferable. Examples of the water-soluble thickener include: polysaccharides (natural polymers) such as cellulose-based thickeners such as hydroxypropyl cellulose, hydroxypropyl methylcellulose, and methyl cellulose, glucomannan, tamarind gum, xanthan gum, guar gum, gum arabic, locust bean gum, and agar; inorganic polymers such as smectite; and acrylic polymers (synthetic polymers) such as sodium polyacrylate and crosslinked sodium polyacrylate. One kind of these may be used alone, or two or more kinds thereof may be used in combination.

Chlorous acid preferably used as a radical generation source is a highly useful radical generation source, but chlorous acid may be decomposed and reduced depending on the coexisting components. According to the studies by the present inventors, it is found that, among the above thickeners, particularly, polysaccharides (natural polymers) such as cellulose-based thickeners such as hydroxypropyl cellulose, hydroxypropyl methylcellulose, and methyl cellulose, glucomannan and tamarind gum, acrylic polymers such as sodium polyacrylate, and smectite hardly cause decomposition of chlorous acid. When at least one selected from the group consisting of chlorous acid, chlorite ions, and chlorites is contained as the radical generation source, these thickeners can be preferably used. Cellulose-based thickeners such as hydroxypropyl cellulose, hydroxypropyl methylcellulose, and methyl cellulose are particularly preferable because they also have excellent long-term thickening stability for the composition.

In the composition according to the present invention, a content of the thickener may be appropriately adjusted in accordance with the kind of the thickener to be used, the desired viscosity of the composition, and the like, and for example, is preferably 0.1 mass% to 20 mass%. When the content of the thickener is 0.1 mass% or more, the handling property is improved because an excessive decrease in the viscosity of the composition is prevented. In addition, when the content of the thickener is 20 mass% or less, the usability can be improved because an excessive increase in the viscosity is prevented.

### (d) Water

The composition according to the present invention contains water as a solvent for dissolving or dispersing the radical generation catalyst, the radical generation source, and the thickener.

Examples of the water include purified water, ion-exchanged water, distilled water, filtered water, and sterilized water.

The composition according to the present invention may contain, as an additive, components other than the radical generation catalyst, the radical generation source, the thickener, and the water described above as long as the effects of the present invention are not impaired.

Examples of the other components include an organic solvent and a pH adjuster.

Examples of the organic solvent include ketones such as acetone, nitrile solvents such as acetonitrile, and alcohol solvents such as ethanol and propylene glycol. One kind of these may be used alone, or two or more kinds thereof may be used in combination.

Examples of the pH adjuster include potassium dihydrogen phosphate, sodium dihydrogen phosphate, dipotassium hydrogen phosphate, disodium hydrogen phosphate, tris(hydroxymethyl)aminomethane, tris(hydroxymethyl)aminomethane hydrochloride, and ethylenediaminetetraacetic acid. One kind of these may be used alone, or two or more kinds thereof may be used in combination.

The composition according to the present invention can be prepared by sequentially mixing and uniformly dissolving the radical generation catalyst (a), the radical generation source (b), the thickener (c), the water (d), and optionally other components.

### (Physical Properties)

The composition according to the present invention may be appropriately adjusted in accordance with an application purpose, and for example, a viscosity at 20°C is preferably 0.005 Pa s to 600 Pa s. When the viscosity at 20°C is 0.005 Pa s or more, the object can be uniformly treated, and the composition is likely to remain on a treated surface. When the viscosity is 600 Pa·s or less, the formulation design is easy, and the production efficiency is also good.

Regarding the composition according to the present invention, when the composition is allowed to stand at 40°C for 2 weeks, a difference between a viscosity at 20°C before standing and a viscosity at 20°C after standing is preferably 50% or less. The difference between the viscosities in the test of 50% or less can maintain the quality of a product because the composition is excellent in temporal stability.

The difference between the viscosities in the test is more preferably 20% or less, and still more preferably 10% or less.

The composition according to the present invention is preferably neutral or alkaline. When the composition is acidic, a radical generation source and a radical generation catalyst rapidly react with each other, and radicals are generated. Therefore, it is difficult to generate radicals stably for a long period of time. In the present invention, states of aqueous radicals (active species) generated in the composition are maintained in a neutral or alkaline environment. When bacteria or viruses as reaction targets are present, the aqueous radicals present in the composition act to eliminate the radicals, but new radicals are generated from the composition. Accordingly, radicals can be generated at the time of necessity.

The pH of the composition is more preferably 7 to 10, and still more preferably 7 to 9.

### (Method For Using Composition)

The composition according to the present invention can be used, for example, as a sterilization agent, a deodorant, an antibacterial agent, an oral care agent, a human body cleansing agent, a disinfectant, or the like.

The method of use may be a common method in the related art. For example, the composition according to the present invention may adhere to an object to be treated, and the composition may be spread directly by hands or by using a tool such as a spoon, a spatula, or a toothbrush.

The member or material to which the composition according to the present invention is applied is not particularly limited, and the composition can be applied to any material such as metal, plastic, glass, wood, paper, cloth, soil, stone, and leaf. Further, the composition can be safely applied to humans and animals.

The target or site to be treated with the composition according to the present invention is not particularly limited, and examples thereof include medical instruments, skin, oral mucosa, hair and the like of humans and animals, articles used in general homes or offices, such as furniture, home appliances, and tableware, and fittings such as door knobs, window glasses, and pipes.

The treatment amount of the composition according to the present invention may be appropriately adjusted in accordance with the treatment target, and for example, when the human skin or oral cavity is treated with the composition, the composition may be used in an amount of about 150 µg/cm² to 0.1 g/cm².

### Examples

Hereinafter, the present invention is further described by Examples, but the present invention is not limited to the following examples. In the following examples, the units "%" and "ppm" are "mass%" and "ppm by mass", respectively.

### (Preparation of Sample Composition)

In accordance with formulations shown in Tables 2 and 3, the components were mixed to prepare sample compositions of Examples 1 to 19.

The sample compositions each had a viscosity of 3 Pa s to 30 Pa s.

In Example 7, 2.0 mass% of propylene glycol was further added to the formulation shown in Table 2, and glucomannan was added to the composition in a state of having been dissolved in propylene glycol.

**[Table 2]**

| Table 2: Examples of formulation of sample composition (unit: mass%) | |
|---|---|
| Component name | Blending amount |
| Sodium chlorite | 0.01 (100 ppm) |
| Benzalkonium chloride | 0.006 (60 ppm) |
| Sodium dihydrogenphosphate dihydrate | 0.01 |
| Disodium hydrogenphosphate dodecahydrate | 0.15 |
| Thickener (see Table 3) | See Table 3 |
| Purified water | Remaining amount |
| Total | 100 |

**[Table 3]**

| Table 3: Kinds and blending amounts of thickeners | | | (unit: mass%) | |
|---|---|---|---|---|
| | Component name | Product name | Maker | Blending amount |
| Example 1 | Hydrophobized hydroxypropyl methylcellulose | Sangelose 90L | Daido Chemical Industry Co., Ltd. | 2.0 |
| Example 2 | Tamarind gum | Glyloid 6C | DSP Gokyo Food & Chemical Co., Ltd. | 3.0 |
| Example 3 | Cationized xanthan gum | Rhaball Gum CX | DSP Gokyo Food & Chemical Co., Ltd. | 2.0 |
| Example 4 | Cationized guar gum | Rhaball Gum CG-8M8 | DSP Gokyo Food & Chemical Co., Ltd. | 2.0 |
| Example 5 | Crosslinked sodium polyacrylate | Rheogic 260H | Toagosei Co., Ltd. | 1.0 |
| Example 6 | Sodium polyacrylate | Cosmedia SP | BASF | 2.0 |
| Example 7 | Glucomannan | Mannangluco HV | Ina Food Industry Co., Ltd. | 1.0 |
| Example 8 | Hydroxypropyl methylcellulose | Benecel K200M | Ashland | 2.0 |
| Example 9 | Hydroxypropyl methylcellulose | Metolose 65SH-30000 | Shin-Etsu Chemical Co., Ltd. | 2.0 |
| Example 10 | Hydroxypropyl cellulose | Klucel H CS | Ashland | 2.0 |
| Example 11 | Hydroxypropyl cellulose | HPC-VH | Nippon Soda Co., Ltd. | 3.5 |
| Example 12 | Hydroxypropyl methylcellulose | Metolose 60SH-10000 | Shin-Etsu Chemical Co., Ltd. | 2.5 |
| Example 13 | Hydroxypropyl methylcellulose | Metolose 90SH-100000 | Shin-Etsu Chemical Co., Ltd. | 2.0 |
| Example 14 | Methyl cellulose | Metolose SM-4000 | Shin-Etsu Chemical Co., Ltd. | 3.0 |
| Example 15 | Smectite | Smecton SWN | Kunimine Industries Co., Ltd. | 5.0 |
| Example 16 | Locust bean gum | MEYPRO LBG FLEUR M-200 | Sansho Co., Ltd. | 2.0 |
| Example 17 | Gum arabic and xanthan gum composite | SOLAGUM AX | Seiwa Kasei Co, Ltd. | 2.0 |
| Example 18 | Agar | Ina Agar Soft | Ina Food Industry Co., Ltd. | 1.0 |
| Example 19 | Tamarind gum | Glyloid 6C | DSP Gokyo Food & Chemical Co., Ltd. | 2.0 |

### <Test Example 1>

### (Confirmation of Appearance of Composition: Examples 1 to 14)

Regarding the sample compositions of Examples 1 to 14, a change in the appearance was observed immediately after preparation. An aqueous solution having the same concentration as that of each of the thickeners described in Table 3 was used as a control, and colors were compared.

A case in which a change in color was not observed was evaluated as "A (good)", and a case in which a change in color was observed due to yellowing or clouding was evaluated as "B (bad)".

The results are shown in Table 4.

### [Table 4]

**Table 4**

| | Thickener used | | Evaluation of appearance |
|---|---|---|---|
| | Component name | Product name | |
| Example 1 | Hydrophobized hydroxypropyl methylcellulose | Sangelose 90L | A |
| Example 2 | Tamarind gum | Glyloid 6C | A |
| Example 3 | Cationized xanthan gum | Rhaball Gum CX | A |
| Example 4 | Cationized guar gum | Rhaball Gum CG-8M8 | A |
| Example 5 | Crosslinked sodium polyacrylate | Rheogic 260H | A |
| Example 6 | Sodium polyacrylate | Cosmedia SP | A |
| Example 7 | Glucomannan | Mannangluco HV | A |
| Example 8 | Hydroxypropyl methylcellulose | Benecel K200M | A |
| Example 9 | Hydroxypropyl methylcellulose | Metolose 65SH-30000 | A |
| Example 10 | Hydroxypropyl cellulose | Klucel H CS | A |
| Example 11 | Hydroxypropyl cellulose | HPC-VH | A |
| Example 12 | Hydroxypropyl methylcellulose | Metolose 60SH-10000 | A |
| Example 13 | Hydroxypropyl methylcellulose | Metolose 90SH-100000 | A |
| Example 14 | Methyl cellulose | Metolose SM-4000 | A |

As shown in Table 4, it was confirmed that there was no change in color in any of the sample compositions and there was no decomposition of sodium chlorite or the like.

### <Test Example 2>

### (Confirmation of Change in Content of Radical Generation Source in Composition: Examples 1 to 19)

Ten grams of each of the sample compositions of Examples 1 to 19 were accurately weighed, and 70 mL of water was added to each of the sample compositions, followed by sufficient stirring. One gram of potassium iodide was added thereto and dissolved. After 20 mL of 3% sulfuric acid was added, the mixture was immediately sealed, allowed to stand in a cold dark place for 15 minutes, and dropped with a 0.01 mol/L sodium thiosulfate solution. A content of sodium chlorite was determined by the following equation (1) (initial content). Sodium chlorite content (ppm) = T × 0.22610 × f × 1000/collection amount of sample composition (g)

In the equation (1), T represents an amount (mL) of the 0.01 mol/L sodium thiosulfate solution required for the dropping, f represents a factor of the 0.01 mol/L sodium thiosulfate solution, and 1 mL of the 0.01 mol/L sodium thiosulfate solution corresponds to 0.22610 mg of sodium chlorite.

Subsequently, after the sample composition was stored for two weeks at 40°C, the content of sodium chlorite was similarly determined (content after storage).

The content (initial content) of sodium chlorite in the sample composition before storage of the composition and the content thereof after storage of the composition are shown in Table 5.

### [Table 5]

**Table 5**

| | Thickener used | | Content of sodium chlorite | |
|---|---|---|---|---|
| | Component name | Product name | Initial content | Content after storage (40°C, 2 weeks) |
| Example 1 | Hydrophobized hydroxypropyl methylcellulose | Sangelose 90L | 101 ppm | 100 ppm |
| Example 2 | Tamarind gum | Glyloid 6C | 83 ppm | 83 ppm |
| Example 3 | Cationized xanthan gum | Rhaball Gum CX | 97 ppm | 98 ppm |
| Example 4 | Cationized guar gum | Rhaball Gum CG-8M8 | 100 ppm | 99 ppm |
| Example 5 | Crosslinked sodium polyacrylate | Rheogic 260H | 100 ppm | 99 ppm |
| Example 6 | Sodium polyacrylate | Cosmedia SP | 100 ppm | 95 ppm |
| Example 7 | Glucomannan | Mannangluco HV | 104 ppm | 100 ppm |
| Example 8 | Hydroxypropyl methylcellulose | Benecel K200M | 103 ppm | 99 ppm |
| Example 9 | Hydroxypropyl methylcellulose | Metolose 65SH-30000 | 105 ppm | 99 ppm |
| Example 10 | Hydroxypropyl cellulose | Klucel H CS | 109 ppm | 110 ppm |
| Example 11 | Hydroxypropyl cellulose | HPC-VH | 105 ppm | 99 ppm |
| Example 12 | Hydroxypropyl methylcellulose | Metolose 60SH-10000 | 105 ppm | 110 ppm |
| Example 13 | Hydroxypropyl methylcellulose | Metolose 90SH-100000 | 105 ppm | 101 ppm |
| Example 14 | Methyl cellulose | Metolose SM-4000 | 105 ppm | 101 ppm |
| Example 15 | Smectite | Smecton SWN | 110 ppm | 98 ppm |
| Example 16 | Locust bean gum | MEYPRO LBG FLEUR M-200 | 110 ppm | 111 ppm |
| Example 17 | Gum arabic and xanthan gum composite | SOLAGUM AX | 103 ppm | 95 ppm |
| Example 18 | Agar | Ina Agar Soft | 102 ppm | 97 ppm |
| Example 19 | Tamarind gum | Glyloid 6C | 104 ppm | 106 ppm |

From the results of Table 5, it was confirmed that the content of sodium chlorite did not significantly change with time, and the reactivity of the thickener with sodium chlorite was low.

### <Test Example 3>

### (Confirmation of Temporal Change in Content of Radical Generation Source: Examples 11 to 15)

Each of the sample compositions of Examples 11 to 15 was stored in a standing manner for three months at 25°C or 40°C. During the storage period, HPLC analysis was performed with time under the following conditions to determine the content of sodium chlorite.

### (HPLC Analysis Conditions)

Analysis device: HPLC manufactured by Shimadzu Corporation
Detector: ultraviolet absorption meter "SPD-20A" (wavelength: 270 nm) manufactured by Shimadzu Corporation
Analysis column: "CAPCELL PAK C18 SG 120" (inner diameter: 4.6 mm, length: 250 mm) manufactured by OSAKA SODA CO., LTD.
Guard column: "CAPCELL PAK C18 SG120 Cartridge Column S5" (inner diameter: 4.0 mm, length: 10 mm) manufactured by OSAKA SODA CO., LTD.
Column temperature: constant temperature around 37°C
Flow rate: 1.0 mL/min
Injection amount: 50 µL
Mobile phase: in 600 mL of methanol were dissolved 400 mL of water, 6.73 g of tetradecyl trimethyl ammonium bromide, and 0.61 g of 2-amino-2-hydroxymethyl-1,3-propane diol, and the pH was adjusted to 7 with phosphoric acid.

The results are shown in FIG. 1. (a) of FIG. 1 shows the results of Example 11, (b) of FIG. 1 shows the results of Example 12, (c) of FIG. 1 shows the results of Example 13, (d) of FIG. 1 shows the results of Example 14, and (e) of FIG. 1 shows the results of Example 15.

From the results of FIG. 1, in all of Examples 11 to 15, there was no large temporal change in the content of sodium chlorite. Although the content of the sodium chlorite tends to decrease with time as the storage temperature increases, it can be determined that the sodium chlorite remains in an amount of 70% or more after 3 months in each case and the long-term storage is enabled.

### <Test Example 4>

### (Confirmation of Temporal Change in Viscosity of Composition: Examples 11 to 15)

Each of the sample compositions of Examples 11 to 15 was stored in a standing manner for three months at 25°C or 40°C. During the storage period, the viscosity was measured with time.

For the measurement of the viscosity, the viscosity that was stable at 20°C was measured using an RB-type viscosity meter ("RB-85H" manufactured by Toki Sangyo Co., Ltd.).

The results are shown in FIG. 2. (a) of FIG. 2 shows the results of Example 11, (b) of FIG. 2 shows the results of Example 12, (c) of FIG. 2 shows the results of Example 13, (d) of FIG. 2 shows the results of Example 14, and (e) of FIG. 2 shows the results of Example 15.

From the results of FIG. 2, in Examples 11 to 14, no large change in the viscosity was observed at both 25°C and 40°C. In Example 15, it was found that there was no large change in the viscosity when stored at 25°C, but the viscosity was increased when stored at 40°C.

From this, it was found that the cellulose-based thickener is excellent in stabilizing the viscosity of the composition.

Although the present invention is described in detail with reference to specific embodiments, it is apparent to those skilled in the art that various changes and modifications can be made without departing from the spirit and scope of the present invention. The present application is based on Japanese Patent Application No. 2021-137556 filed on August 25, 2021, the contents of which are incorporated herein by reference.

## Claims

1. A composition comprising a radical generation catalyst (a), a radical generation source (b), a thickener (c), and water (d).

2. The composition according to claim 1, wherein the radical generation catalyst is a Lewis acid having a Lewis acidity of 0.4 eV or more.

3. The composition according to claim 1 or 2, wherein the radical generation catalyst contains an ammonium salt represented by the following chemical formula (I): 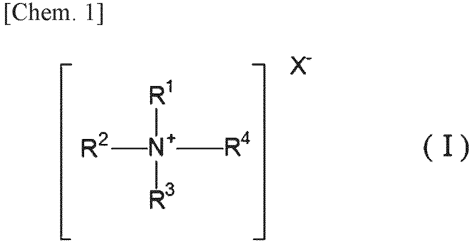 wherein in the chemical formula (I), R¹, R², R³, and R⁴ are the same or different from each other, each represents a hydrogen atom or an alkyl group, and may contain an ether bond, a carbonyl group, an ester bond, an amide bond, or an aromatic ring, and X⁻ represents an anion.

4. The composition according to claim 3, wherein the ammonium salt represented by the chemical formula (I) is an ammonium salt represented by the following chemical formula (II): 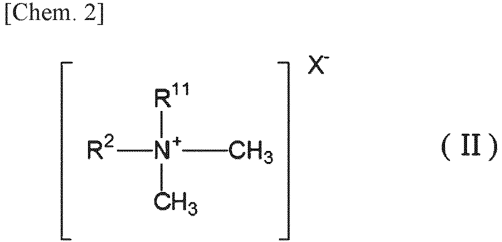 wherein in the chemical formula (II), R¹¹ represents an alkyl group having 5 to 40 carbon atoms and may contain an ether bond, a carbonyl group, an ester bond, an amide bond, or an aromatic ring, and R² and X⁻ are the same as those in the chemical formula (I).

5. The composition according to claim 4, wherein the ammonium salt represented by the chemical formula (II) is an ammonium salt represented by the following chemical formula (III): 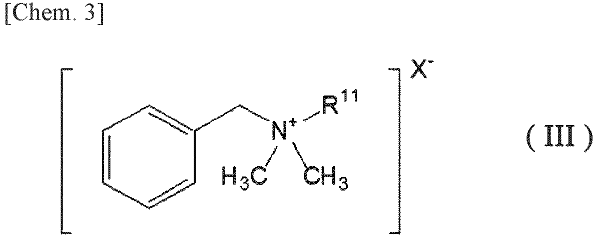 wherein in the chemical formula (III), R¹¹ and X⁻ are the same as those in the chemical formula (II).

6. The composition according to claim 1, wherein the radical generation source is at least one selected from the group consisting of a halous acid, a halite ion, and a halite.

7. The composition according to claim 1, which is neutral or alkaline.
